(19) Europäisches Patentamt
European Patent Office
Office européen des brevets

(11) Numéro de publication : **0 209 561**
**B1**

(12) # FASCICULE DE BREVET EUROPÉEN

(45) Date de publication du fascicule du brevet : **11.10.89**

(51) Int. Cl.$^4$ : **A 41 B 13/02**, A 61 L 15/00, A 61 F 13/18

(21) Numéro de dépôt : **86900784.9**

(22) Date de dépôt : **17.01.86**

(86) Numéro de dépôt international : **PCT/FR 86/00012**

(87) Numéro de publication internationale : **WO/8604219 (31.07.86 Gazette 86/17)**

(54) **PRODUIT D'HYGIENE COMPORTANT UN MATELAS ABSORBANT MUNI D'UN INDICATEUR D'HUMIDITE ET PROCEDE DE FABRICATION.**

(30) Priorité : **17.01.85 FR 8500672**

(43) Date de publication de la demande : **28.01.87 Bulletin 87/05**

(45) Mention de la délivrance du brevet : **11.10.89 Bulletin 89/41**

(84) Etats contractants désignés : **AT BE CH DE GB IT LI NL**

(56) Documents cités :
**EP–A– 0 140 560**
**FR–A– 2 438 475**
**FR–A– 2 486 112**
**US–A– 4 192 311**

(73) Titulaire : **PEAUDOUCE**
**59, Rue de la Vignette**
**F-59126 Linselles (FR)**

(72) Inventeur : **COURTRAY, Franck**
**110 ter, rue de la Vignette**
**F-59126 Linselles (FR)**

(74) Mandataire : **Casalonga, Axel et al**
**BUREAU D.A. CASALONGA - JOSSE Morassistrasse 8**
**D-8000 Munich 5 (DE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 209 561 B1

## Description

La présente invention est relative à un produit d'hygiène comportant un matelas absorbant muni de moyens fournissant une indication sur le degré d'humidité du matériau absorbant.

Les produits d'hygiène peuvent être constitués par des produits jetables pour l'hygiène et les pansements tels que les couches pour enfants de bas âge, les couches culottes fréquemment connues sous l'appellation de « change complet » pouvant comporter ou non des moyens élastiques au voisinage de l'entrejambe afin d'améliorer les caractéristiques d'étanchéité. Ces produits peuvent également être utilisés par des personnes adultes incontinentes. Les matelas absorbants munis d'un indicateur d'humidité conformes à l'invention peuvent également être utilisés pour des garnitures périodiques, des pansements et d'une manière générale pour tous articles d'hygiène destinés à absorber l'humidité telle que celle des fluides biologiques et notamment celle qui résulte de l'urine ou du sang.

La demanderesse a déjà proposé dans sa demande de brevet français n° 80 14863 un matelas absorbant comportant un indicateur d'humidité constitué par un support allongé disposé en contact avec le matelas absorbant sur toute la longueur du matelas et recouvert ou imprégné d'une matière colorante sensible à l'humidité. L'apparition de l'humidité entraînait la mise en solution du colorant dans l'urine et donc l'apparition d'une ligne colorée de largeur irrégulière due à la diffusion du colorant autour du support.

De tels dispositifs présentent cependant l'inconvénient de nécessiter l'utilisation de colorants solubles dans l'eau ou l'urine et susceptibles d'entraîner la diffusion du colorant autour du support initial.

Il a également été proposé d'autres dispositifs indicateurs d'humidité consistant notamment, comme indiqué dans les brevets américains 3 004 895 et 3 675 654, à recouvrir le matelas absorbant d'une couche pour bébé d'un produit sensible à l'ammoniac ou à l'humidité. On utilise dans ce but un produit pulvérulent recouvrant en couche mince la totalité ou la majeure partie de l'une des faces du matelas absorbant afin de permettre un effet désodorisant évitant un dégagement d'ammoniac ou permettant de détecter l'humidité du matelas absorbant par une modification de couleur du colorant pulvérulent recouvrant le matelas.

La présente invention permet de résoudre ce problème de façon simple en évitant les inconvénients des moyens utilisés antérieurement, grâce à l'utilisation des polymères superabsorbants colorés dans la masse. La demanderesse a découvert qu'il était possible de réaliser de façon simple un matelas absorbant comportant des moyens susceptibles d'indiquer le degré d'humidité dudit matelas sans nécessiter soit des colorants changeants de couleur ou soit des colorants se solubilisant dans le liquide ou le fluide à l'origine de l'humidité.

En effet, grâce à l'application de tels polymères superabsorbants colorés, l'indication d'une humidité résulte d'une visualisation améliorée du fait du gonflement du polymère superabsorbant coloré dans la masse.

Il est à noter que l'indication d'humidité n'est donc plus tributaire d'une réaction chimique entraînant la modification d'une couleur ou bien des caractéristiques de solubilisation dans le milieu à l'origine de l'humidité.

L'utilisation de ce type de polymère permet également d'améliorer de façon considérable les conditions de fabrication des matelas absorbants comportant des moyens susceptibles d'indiquer le degré d'humidité de ces derniers.

L'invention a pour objet un produit d'hygiène comportant au moins un indicateur d'humidité constitué par un polymère superabsorbant coloré dans la masse, comme décrit dans la revendication 1.

Un autre objet de l'invention est constitué par un procédé de fabrication d'un matelas absorbant comportant des moyens susceptibles d'indiquer le degré d'humidité dudit matelas, comme décrit dans la revendication 6.

D'autres objets apparaîtront à la lecture de la description des exemples qui suivent.

Le produit d'hygiène destiné à absorber l'humidité due aux fluides biologiques et en particulier l'urine et le sang conforme à l'invention comprend un matelas absorbant disposé sur une feuille imperméable translucide ou partiellement transparente, ledit matelas absorbant ou ladite feuille imperméable comportant sous forme de lignes continues, de points, de bande ou de dessins représentant des motifs divers, un élastomère superabsorbant coloré dans la masse appliqué soit sur la feuille imperméable du côté du matelas absorbant, soit sur toute feuille perméable en contact avec le matelas absorbant mais visible de l'extérieur au cours de l'utilisation.

On appelle « imperméable » une feuille imperméable aux fluides biologiques mais éventuellement perméable à l'air une feuille perméable étant perméable aux fluides biologiques.

On appelle « polymères superabsorbants » des polymères non solubles dans l'eau ayant la propriété d'absorber de l'eau pure dans des proportions de plus de 10 fois leur poids et présentant de bonnes propriétés de rétention de l'eau même sous pression. Il est entendu que dans l'application à des produits d'hygiène ces polymères doivent être non toxiques.

Les polymères superabsorbants sont choisis notamment parmi les dérivés d'amidon greffé, les dérivés de carboxyméthylcellulose, les dérivés d'acide acrylique ou méthacrylique, d'alcool vinylique ou d'acide ou d'anhydride maléique sous forme de leurs sels tels que les sels de métaux alcalins ou alcalino-terreux comme de sodium ou de potassium.

Les polymères préférés ont un motif acrylate dans leur structure et sont choisis notamment parmi les homopolymères ou copolymères acryliques synthétiques ou des polysaccharides greffés avec des monomères acryliques.

Des polymères superabsorbants préférés de ce type sont des polymères acryliques comportant des groupes acide ou sel ou des groupes amide et acide éventuellement salifiés tels que ceux répondant à la formule :

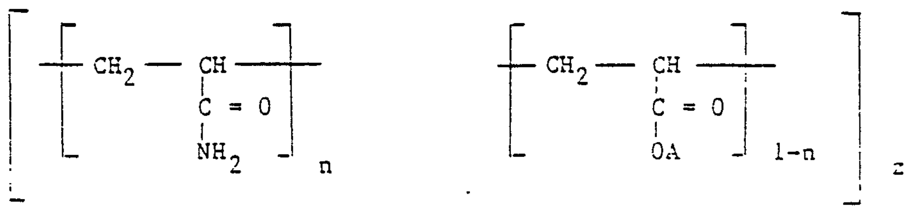

où A est un métal alcalin tel que sodium ou potassium, n est compris entre 0,5 et 0,9, z est le nombre d'unités entre les ponts dûs à la réticulation.

De tels polymères sont décrits notamment dans les brevets américains 3 686 024, 4 192 727, 3 229 769.

Les polysaccharides greffés avec des monomères acryliques préférés sont ceux comportant des groupements hydrophiles greffés sur le polysaccharide tel que carboxyle, sulfonique, hydroxyle, amide, amino, ammonium quaternaire et leurs produits d'hydrolyse.

Les chaînes hydrophiles peuvent être représentées par la formule :

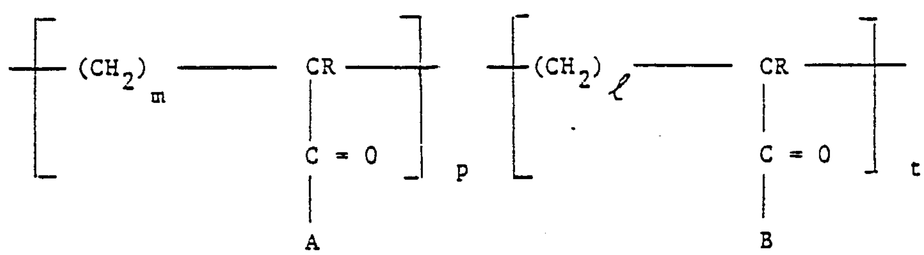

attachées au squelette de la cellulose ou de l'amidon. Les groupes A et B désignent OH, O alkyl inférieur, métal alcalin, ammonium, amine, R désigne hydrogène ou alkyle inférieur, n désigne 0 ou 1, ℓ désigne un nombre entier de 1 à 4, p et t désignent un nombre entier de 0 à 5 000, et p + t est égal à au moins 500.

De tels polymères et leurs procédés de fabrication sont décrits dans le brevet américain 4 028 290.

D'autres polymères superabsorbants sont les carboxyméthylcellulose réticulées décrites dans le brevet américain 2 639 239, des poly (alkylène oxyde) réticulés décrits dans les brevets US 3 898 143, 3 957 605, 3 264 202, 3 956 224 et leurs mélanges avec des gommes.

On peut citer les copolymères d'amidon et d'acrylonitrile greffés et saponifiés, les copolymères d'amidon et d'acide acrylique greffés ; les polymères dérivés d'acide acrylique ou méthacrylique réticulés ainsi que leurs copolymères avec des monomères non solubles dans l'eau ; les polymères dérivés d'acide acrylique peuvent être constitués également par des copolymères avec des monomères divinyliques tels que le divinylbenzène et le 1 ou 2-polybutadiène.

Ces polymères peuvent être préparés par polymérisation avec des initiateurs radicalaires choisis parmi les persulfates ou peroxydes ou de présence d'agents de surface sous forme d'esters, de dérivés de polymères colloïdaux tels que ceux dérivés des acides gras du sorbitan.

Ces polymères peuvent également avoir subi des traitements de façon à améliorer encore leur propriété d'absorption par polymérisation avec de l'hydroxyéthyl cellulose, avec le 2-acrylamide, l'acide 2-méthylpropane sulfonique, le 2-hydroxyéthyl acrylate ou méthacrylate.

D'autres polymères utilisables comme polymères superabsorbants sont les copolymères d'anhydride et d'acides cycliques tels que l'anhydride maléique copolymérisé avec de l'alcool vinylique ou avec des dérivés éthyléniques tels que par exemple l'isobutylène.

Des polymères superabsorbants utilisables conformes à l'invention peuvent résulter de la neutralisation du produit d'addition d'acide thioglycolique sur des copolymères blocs styrène/butadiène ou sur des polybutadiène/styrènes greffés à double liaison insaturée. On peut également utiliser des polymères dérivés d'acide ou d'anhydride maléique réticulés avec des dérivés époxylés polyhydriques tels que l'éther de diglycidyle glycérine ou une amine polyhydroxylée telle que la tétra-éthylène pentamine.

On peut également utiliser selon une forme de réalisation préférentielle des mélanges de ces polymères superabsorbants avec des élastomères sous forme de mélanges fusibles à chaud tels que des mélanges avec des copolymères éthylène/acétate de vinyle.

Ces polymères superabsorbants ou les mélanges d'élastomères et de polymères superabsorbants

sont de préférence colorés dans la masse et peuvent notamment avoir des couleurs différentes choisies suivant la taille du produit d'hygiène.

Les lignes, points, bandes ou dessins de polymères superabsorbants sont positionnés dans le produit d'hygiène à proximité du matelas absorbant et du côté du produit d'hygiène visible de l'extérieur après application. Ces moyens d'indication de l'humidité sont en particulier appliqués sur la feuille imperméable extérieure du côté de la feuille tournée vers le matelas absorbant ou alors sur la feuille perméable directement placée en contact avec le matelas absorbant qui est généralement en non tissé, en tissu, en feutre, en ouate de cellulose, etc.

Cet indicateur d'humidité peut être fixé soit par collage sur les supports susmentionnés soit par calendrage, par extrudage, ou alors par impression dans le cas notamment des motifs lors de l'utilisation de l'élastomère superabsorbant. L'intérêt principal de ces indicateurs d'humidité réside dans le fait qu'ils peuvent être facilement appliqués et que le colorant ne diffuse pas.

A titre d'exemple de matériaux superabsorbants utilisables on peut citer les produits vendus sous la dénomination « KIGEL » par la Société KURARAY ISOPRENE Chemical Corporation à base d'un copolymère d'anhydride maléique et d'isobutylène. On peut également citer les produits vendus sous la dénomination « ARASORB » qui sont des dérivés d'acide acrylique, les produits vendus sous la dénomination « AQUA-KEEP » par la Société SEITETSU Chemical Industry Corporation qui est un dérivé d'acide polyacrylique, les produits vendus sous la dénomination « STASORB » par A.E. STALEY, « FAVORSAB » par STOCKHAUSEN, « PERMASORB » par NATIONAL STARCH, « AKUCELL » par ENKA INDUSTRIES, SGP ou SPC 502S par HENKEL.

Un procédé de fabrication préféré d'un produit d'hygiène conforme à l'invention comprend la succession d'étapes suivantes :

on introduit dans un premier temps un mélange d'élastomère et de polymère superabsorbants teint dans la masse, fusible à chaud (hot melt) dans une extrudeuse. On applique à chaud par le moyen de l'extrudeuse ce mélange sur la feuille de polyéthylène sur le côté destiné à être en contact avec la nappe de produit absorbant. On applique ensuite une nappe de produit absorbant qui peut être par exemple de la pulpe de cellulose broyée connue sous l'appellation de « fluff » de cellulose. On recouvre ensuite en continu la nappe de produit absorbant éventuellement par une bande supérieure perméable et on découpe finalement en élément individuel la bande continue ainsi obtenue.

Lorsqu'on n'utilise pas un mélange fusible à chaud on peut encoller le polymère superabsorbant par exemple par un produit d'encollage tel que de la fécule, de l'amidon, un dérivé cellulosique tel que la carboxyméthyl cellulose ou l'alcool polyvinylique ou une colle fusible à chaud qui donnent de bons résultats.

L'invention sera mieux comprise à l'étude de la description détaillée faite à titre d'exemple nullement limitatif d'un mode de réalisation particulier des produits conformes à l'invention.

Le change complet comprend une feuille imperméable souple extérieure constituant lors de l'utilisation de revêtement extérieur du change complet. Cette feuille imperméable souple est avantageusement réalisée en polyéthylène semi-transparent. Elle présente une forme générale rectangulaire et comporte au voisinage de l'axe de symétrie transversal dans la zone de l'entrejambe deux échancrures latérales pour le passage des jambes du bébé. Chaque échancrure comprend une partie rectiligne sensiblement parallèle à l'axe longitudinal de la couche-culotte et deux parties de jonction qui relient la partie rectiligne et des bords respectifs avant et arrière de la feuille imperméable. Sur la face interne de la feuille imperméable dans la zone médiane longitudinale de cette dernière sont disposés dans un mode de réalisation des filets de polymère superabsorbant colorés dans la masse. Sur la feuille imperméable est disposé un matelas absorbant qui se trouve intercalé entre la bande inférieure en ouate de cellulose qui est en contact avec la feuille imperméable et les filets et la nappe de produit absorbant en fluff de cellulose laquelle est à son tour recouverte par la bande supérieure en ouate de cellulose.

Selon une autre variante de l'invention le polymère superabsorbant est appliqué sous forme de pointillés sur la bande perméable par exemple en ouate de cellulose en contact avec la feuille imperméable. Dans les deux cas, le polymère superabsorbant est en contact direct avec l'humidité que le « fluff » a pour fonction d'absorber.

Deux éléments élastiques rectilignes et parallèles à l'axe longitudinal de la couche culotte sont disposés de chaque côté de bords latéraux du matelas absorbant et sont fixés par exemple par collage sur la face interne de la feuille imperméable au moins en regard de la zone de l'entrejambe de façon à exercer une traction principalement dans la zone de l'entrejambe dans une direction sensiblement parallèle à l'axe longitudinal de la couche culotte. Une feuille perméable à l'intérieur est en outre fixée sur toute la périphérie de la face interne de la feuille imperméable de façon à envelopper convenablement le matelas absorbant et les éléments élastiques. Cette feuille perméable peut être réalisée de façon classique en un matériau non tissé.

Pour refermer les bords avant et arrière de façon à former la culotte le bord arrière comporte deux languettes adhésives montées sur les bords de la feuille imperméable et la feuille perméable. La partie interne de chacune des languettes comporte un revêtement adhésif de sorte qu'il est possible au moment de l'utilisation de décoller l'un des rabats adhésifs qui peut venir se fixer sur la face interne du bord avant de la feuille imperméable.

Une fois la couche culotte mise en place sur le bébé, l'urine pénétrant à travers la feuille intérieure est

retenue par le matelas absorbant et vient agir sur le polymère superabsorbant coloré dans la masse qui augmente de volume en l'espace de quelques minutes, ce qui permet de visualiser le degré de l'humidité. Au contraire tant que le coussin est sec la ligne colorée ou les points colorés réalisés par des filets ou les points de polymère superabsorbant colorés dans la masse sont très fins et presque invisibles.

L'indicateur d'humidité du matelas absorbant est donc perceptible par transparence lorsque la couche culotte est utilisée du milieu de la ceinture avant au milieu de la ceinture arrière et peut donc être vu quelle que soit la position du bébé debout, couché. La longueur et la largeur ou la surface de la partie mouillée permettent de déterminer facilement l'importance de l'humidité du coussin absorbant.

Bien que l'invention soit illustrée au moyen d'une couche culotte pour bébé on comprend qu'elle pourrait être également appliquée sans modifications notables à d'autres articles d'hygiène et notamment des garnitures périodiques.

Par ailleurs, bien que deux types de filets de polymère absorbant aient été illustrés, on comprendra que l'on puisse envisager de disposer d'autres formes sur la feuille imperméable ou sur une feuille perméable en contact direct avec la nappe absorbante.

## Revendications

1. Produit d'hygiène destiné à absorber l'humidité provenant des fluides biologiques, en particulier l'urine et le sang comportant un matelas absorbant disposé sur une feuille imperméable caractérisé par le fait que ledit matelas absorbant est associé à un élément sensible à l'humidité jouant le rôle d'indicateur d'humidité constitué par un polymère superabsorbant coloré dans la masse appliqué sous forme de lignes, de points, de bandes ou dessins soit sur la feuille imperméable du côté dudit matelas absorbant soit sur toute feuille perméable en contact avec ledit matelas absorbant et visible de l'extérieur au cours de l'utilisation.

2. Produit d'hygiène selon la revendication 1, caractérisé par le fait que ledit polymère absorbant est constitué par un mélange fusible à chaud d'un élastomère et d'un polymère superabsorbant coloré dans la masse.

3. Produit d'hygiène selon la revendication 1 ou 2, caractérisé par le fait que le polymère superabsorbant est choisi parmi les dérivés d'amidon greffé, les dérivés de carboxyméthylcellulose, les dérivés d'acide acrylique ou méthacrylique, d'alcool vinylique, d'acide ou d'anhydride maléique sous forme de leurs sels.

4. Produit selon l'une quelconque des revendications 1 à 3, caractérisé par le fait que le polymère superabsorbant est un polyacrylate de sodium réticulé, un copolymère d'anhydride maléique et d'isobutylène.

5. Produit selon l'une quelconque des revendications 1 à 4, caractérisé par le fait que l'on utilise un mélange fusible à chaud d'élastomère et de polymère superabsorbant.

6. Procédé de fabrication d'un produit d'hygiène destiné à absorber l'humidité provenant des fluides biologiques, caractérisé par le fait :

— que l'on applique sur une feuille imperméable des lignes, points, bandes ou dessins de polymère superabsorbant colorés dans la masse ;

— que l'on dépose en continu sur la feuille imperméable ainsi revêtue une nappe d'un produit absorbant ;

— que l'on recouvre si nécessaire en continu la nappe de produit absorbant par une bande supérieure perméable ; et

— que l'on découpe en élément individuel la bande continue ainsi obtenue.

## Claims

1. Hygiene product intended to absorb the wetness of biological fluids, in particular urine and blood, comprising an absorbent padding arranged on an impermeable sheet, characterized in that the said absorbent padding is associated with an element which is sensitive to wetness and plays the part of wetness indicator, constituted by a superabsorbent bulk-coloured polymer applied in the form of lines, dots, bands or patterns either on the impermeable sheet, on the side of the said absorbent padding, or on any permeable sheet which is in contact with the said absorbent padding and visible from the exterior during use.

2. Hygiene product according to Claim 1, characterized in that the said absorbent polymer is constituted by a hot-melting mixture of an elastomer and a super-absorbent bulk-coloured polymer.

3. Hygiene product according to Claim 1 or 2, characterized in that the superabsorbent polymer is chosen from the derivatives of grafted starch, the derivatives of carboxymethylcellulose and the derivatives of acrylic or methacrylic acid, of vinyl alcohol or of maleic acid or maleic anhydride in the form of their salts.

4. Product according to any one of Claims 1 to 3, characterized in that the superabsorbent polymer is a crosslinked sodium polyacrylate or a copolymer of maleic anhydride and isobutylene.

5. Product according to any one of Claims 1 to 4, characterized in that a hot-melting mixture of elastomer and superabsorbent polymer is used.

6. Process for manufacture of a hygiene product intended to absorb the wetness of biological fluids, characterized in that :

— lines, dots, bands or patterns of superabsorbent bulk-coloured polymer are applied to an impermeable sheet ;

— a layer of an absorbent product is continuously placed on the impermeable sheet which has been thus coated ;

— the layer of absorbent product is, if necessary, covered continuously with an upper permeable band ; and

— the continuous band thus obtained is cut into individual elements.

**Patentansprüche**

1. Hygieneartikel, der dazu bestimmt ist, die Feuchtigkeit, die von biologischen Flüssigkeiten, insbesondere Harn und Blut herrührt, zu absorbieren, mit einem saugfähigen Kissen, das auf einer undurchlässigen Folie angeordnet ist, dadurch gekennzeichnet, daß das saugfähige Kissen einem feuchtigkeitsempfindlichen Element zugeordnet ist, das als Feuchtigkeitsanzeiger dient und von einem stark absorbierenden, in der Masse gefärbten Polymeren gebildet wird, das entweder in Form von Linien, Punkten, Streifen oder Mustern auf der undurchlässigen Folie auf der Seite des saugfähigen Kissens oder auf der gesamten, durchlässigen Folie angebracht ist, die mit dem saugfähigen Kissen in Berührung ist und während des Gebrauches von außen sichtbar ist.

2. Hygieneartikel nach Anspruch 1, dadurch gekennzeichnet, daß das absorbierende Polymer eine heißschmelzbare Mischung eines Elastomeren und eines stark absorbierenden in der Masse gefärbten Polymeren ist.

3. Hygieneartikel nach Anspruch 1 oder 2, dadurch gekennzeichnet, daß das stark absorbierende Polymer ausgewählt ist aus veredelten Stärkederivaten, Carboxymethylcellulosederivaten, Acrylsäure- oder Metacrylsäurederivaten, Vinylalkohol, Maleinsäure oder Maleinsäuranhydrid in Form ihrer Salze.

4. Artikel nach einem der Ansprüche 1 bis 3, dadurch gekennzeichnet, daß das stark absorbierende Polymer ein vernetztes Natriumpolyacrylat, ein Kopolymer aus Maleinsäureanhydrid und Isobutylen ist.

5. Artikel nach einem der Ansprüche 1 bis 4, dadurch gekennzeichnet, daß man eine heißschmelzbare Mischung des Elastomeren und des stark absorbierenden Polymeren verwendet.

6. Verfahren zur Herstellung eines Hygieneartikels, der dazu dient, Feuchtigkeit, die von biologischen Flüssigkeiten herrührt, zu absorbieren, dadurch gekennzeichnet :

— daß man auf eine undurchlässige Folie Linien, Punkte, Streifen oder Muster des in der Masse gefärbten, stark absorbierenden Polymeren aufbringt,

— daß man auf die so bedeckte undurchlässige Folie fortlaufend eine Einlage aus einem saugfähigen Produkt anbringt,

— daß man, falls notwendig, die Einlage des absorbierenden Produktes fortlaufend mit einem oberen, durchlässigen Band bedeckt ; und

— daß man das so erhaltene, fortlaufende Band in einzelne Teile zerschneidet.